# FASCICULE DE BREVET EUROPEEN

(11) **EP 2 672 961 B1**
(45) Date de publication et mention de la délivrance du brevet: **06.06.2018**
(21) Numéro de dépôt: 12708564.5
(22) Date de dépôt: 03.02.2012
(51) Int. Cl.: A61K 9/50, A61K 31/19, A61K 9/00

(54) **GRANULES EFFERVESCENTS D'ACIDE GAMMA-HYDROXYBUTYRIQUE**
BRAUSENDES GAMMA-HYDROXYBUTTERSÄURE-GRANULAT
EFFERVESCENT GAMMA-HYDROXYBUTYRIC ACID GRANULES

(30) Priorité: 11.02.2011 FR 1100433
(43) Date de publication de la demande: 18.12.2013
(73) Titulaire: Debregeas Et Associes Pharma, 75008 Paris (FR)
(72) Inventeur: SUPLIE, Pascal, F-27400 Montaure (FR); LECOUSTEY, Sylvie, F-28500 Mezieres en Drouais (FR)
(74) Mandataire: Gallochat, Alain
(86) Numéro de dépôt international: PCT/FR2012/000046
(87) Numéro de publication internationale: WO 2012/107652

(56) Documents cités:
- EP-A2- 1 273 301
- WO-A1-2010/055260
- WO-A1-2010/055268
- WO-A2-2011/018583
- US-A1- 2006 210 630
- ANONYMOUS: "Rohypnol", CONNECTICUT CLEARINGHOUSE - A PROGRAM OF WHEELER CLINIC, , 1 janvier 2003 (2003-01-01), pages 1-2, XP002628783, Extrait de l'Internet: URL:http://www.ctclearinghouse.org/topics/ customer-files/rohypnol.pdf [extrait le 2011-03-17]

## Description

La présente invention a pour objet des granulés d'acide gamma-hydroxybutyrique, ainsi que des compositions pharmaceutiques les contenant. L'acide gamma-hydroxybutyrique (GHB ou oxybate de sodium) est un métabolite naturel présent au niveau du cerveau des mammifères ; sa structure chimique est proche du neurotransmetteur acide gamma-aminobutyrique (GABA), d'ou son activité sur le système nerveux central.

Ce principe actif a été utilisé comme anesthésiant général et comme hypnotique dans le traitement de l'insomnie. Il est actuellement surtout utilisé dans certains troubles du sommeil, tels que la catalepsie chez les patients narcoleptiques sous le nom commercial Xyrem®, à raison de deux prises nocturnes de soluté. Dans ce cadre, le patient prend une dose initiale au coucher et doit se lever environ quatre heures après pour une deuxième prise nocturne.

Le GHB est très soluble, hygroscopique et fortement alcalin. Les doses thérapeutiques sont très élevées. En effet, dans le traitement de la narcolepsie, les doses utilisées vont de 4,5 g à 9 g de principe actif par jour.

Par ailleurs, le GHB est fréquemment utilisé comme drogue récréative dans différents objectifs : désinhibition, détente, recherche de performances physique ou sexuelle.

L'ensemble de ces caractéristiques démontre l'intérêt de disposer d'une forme à libération prolongée de ce principe actif. De nombreux travaux ont été menés en ce sens. Ainsi, on peut relever dans l'art antérieur différentes formulations orales à libération prolongée sans toutefois qu'aucune n'apporte une réponse complète à l'ensemble de cette problématique complexe.

US 5 594 030 décrit des compositions pharmaceutiques se présentant sous forme de granulés ou comprimés contenant le GHB dans une matrice cellulosique ; ces préparations pharmaceutiques présentent une dissolution sur une durée de 7 à 8 heures.

EP 0 635 265 décrit des compositions pharmaceutiques à libération contrôlée à base de sel d'acide gamma-hydroxybutyrique constituées d'un noyau sous forme de granulés ou comprimés comprenant le principe actif dispersé dans une matrice cellulosique. On obtient ainsi une forme à libération contrôlée à l'aide de diffusion au travers de membranes visant une libération dans le haut du tube digestif du principe actif.

La demande US2006/210630 décrit des compositions pharmaceutiques à libération contrôlée de GHB constituées de particules comprenant le principe actif comme noyau. Ces particules sont ensuite recouvertes d'une couche de protection, celle-ci étant elle-même recouverte d'une seconde couche à libération entérique.

Il est donc connu de l'état de la technique des particules immédiates ou gastroprotégées pouvant être utilisées seules ou en combinaison.

La demande PCT WO 2010/055260 décrit un granulé comprenant un coeur solide sur lequel est supporté GHB, éventuellement du carbonate de calcium, du PVP, d'un colorant, ledit granulé étant enrobé par une couche d'hypromellose puis de l'hypromellose phtalate.

Le granulé décrit dans cette demande permet de réduire la dose journalière et le nombre de prises quotidiennes, en augmentant la demi-vie apparente et la biodisponibilité du principe actif. Par ailleurs, il est doté de moyens qui réduisent les détournements d'utilisation.

Dans le granulé décrit dans cette demande de brevet, l'agent d'enrobage représente au moins 24% en poids par rapport au poids du granulé et le carbonate de calcium est un agent de masse et n'agit pas comme agent générateur de gaz.

Enfin, et c'est là un point essentiel, le coeur solide du dit granulé représente 11,48% en poids par rapport au poids total du granulé.

La demande PCT WO 2011/018583 décrit une composition destinée à éviter le mésusage des médicaments et a un objectif différent de celui de la composition selon la présente invention, outre le fait qu'elle décrit des constituants différents de ceux faisant l'objet de la présente invention.

Il est important de rappeler que la problématique du GHB est complexe et multiple: en effet, le GHB est extrêmement soluble et après administration orale, la biodisponibilité absolue n'est que de 25%. Les temps moyens d'atteinte des taux plasmatiques vont de 30 minutes à 2 heures, l'absorption étant très variable d'un individu à l'autre.

L'élimination se fait essentiellement par métabolisation en dioxyde de carbone avec une demi-vie allant de 30 minutes à une heure. Cependant l'absorption semble être de capacité limitée et régio-spécifique, et plus particulièrement située très haut dans le tube digestif (Palatini et al, European Journal of Clinical Pharmacology, 1993).

L'ensemble des formulations décrites dans l'art antérieur mentionne des formes à libération prolongée et plus particulièrement des formes gastroprotégées, c'est-à-dire pour lesquelles la libération du GHB se fera à la sortie de l'estomac, ceci grâce à l'utilisation de polymère pH-dépendant (dissolution du polymère à pH 5,5 ou 6).

On obtient, dans ce cas et également dans le cas des matrices, au niveau physiologique, un décalage dans le temps de l'absorption du GHB. Ceci n'est toutefois pas complètement satisfaisant car le temps de présence au niveau des sites préférentiels d'absorption reste limité et plus l'effet de libération prolongée est important, moins la concentration effective sanguine est importante.

Ce type de formulation ne convient pas au traitement de pathologies nécessitant la mise à disposition très rapide *in vivo* de GHB telles que notamment la narcolepsie ou le sevrage de l'alcoolisme.

La solution apportée par la présente invention permet de répondre à cette problèmatique.

Ainsi, la présente invention a pour but de fournir une nouvelle forme galénique à base d'acide gamma-hydroxybutyrique ou d'un de ses sels (notamment sodium) permettant de contourner les inconvénients susmentionnés.

La présente invention a pour but de fournir une nouvelle forme galénique à base d'acide gamma-hydroxybutyrique ou d'un de ses sels permettant d'augmenter le temps de résidence du principe actif dans l'estomac.

La présente invention concerne un granulé comprenant un coeur solide sur lequel est supporté un principe actif, ledit principe actif étant choisi parmi l'acide gamma-hydroxybutyrique ou l'un de ses sels pharmaceutiquement acceptables, ledit granulé se composant de :
- 15-25% de coeur solide,
- 50-60% de principe actif
- 5-15% de bicarbonate de sodium, générateur de gaz
- 2-18% d'aluminometasilicate de magnésium, diluant
- 3-10% de gomme laque, liant
- 3-6% de membrane d'enrobage

Le caractère alcalin du principe actif a donc été renforcé par l'ajout de sels alcalins mélangés au principe actif et inclus au coeur du granule actif. On obtient alors, en plongeant les granulés ainsi constitués dans un liquide acide, une rémanence des granulés à la surface du liquide. L'utilisation de l'agent alcalin (susceptible de générer un dégagement gazeux) en combinaison avec un enrobage permet alors de moduler la réaction avec ce même milieu et finalement d'assurer un temps de présence prolongé au niveau des sites d'absorption.

La membrane des granulés selon la présente invention permet au principe actif de diffuser lentement puis de réagir avec le milieu stomacal et finalement d'assurer un temps de présence prolongé au niveau des sites d'absorption.

La présente invention concerne donc une forme multiparticulaire de granules ou de granulés. Ces granulés sont constitués d'un coeur solide lui-même constitué ou non d'un support solide, sur lequel est déposé un mélange de principe actif et différents excipients. Ces excipients sont choisis parmi les composés susceptibles de générer un dégagement gazeux (agents alcalins) et parmi les diluants. Le granulé obtenu est ensuite entouré d'une membrane, qui permettra de relâcher progressivement le principe actif et les adjuvants nécessaires au maintien de la forme en surface du bol stomacal. Préférentiellement, la membrane est choisie parmi les excipients d'enrobage indépendants du pH et plus préférentiellement la gomme laque.

La présente invention consiste à fournir des formulations pharmaceutiques comprenant l'utilisation de plusieurs artifices galéniques afin de rendre impossible chacune des techniques rencontrées lors des utilisations détournées. Ces formulations, à base des granulés susmentionnés, se présentent sous forme monolithique (comprimés) ou multiparticulaire. Lesdits granulés de l'invention comportent donc plusieurs couches de composition différente présentant chacune une fonctionnalité particulière.

L'expression "granulé" désigne une préparation constituée de grains solides secs, formant chacun un agrégat de particules de poudre d'une solidité suffisante pour permettre diverses manipulations.

Du point de vue physique, les granulés sont des agrégats de particules de poudres diverses cristallisées ou amorphes.

Les granulés de la présente invention sont notamment destinés à une administration par voie orale, et plus particulièrement à être avalés tels quels.

Les granulés de la présente invention présentent une structure caractéristique de type coeur-écorce, le coeur n'étant pas de même nature que les composés formant l'écorce.

Ainsi, ces granulés présentent une structure multicouche. En effet, le principe actif est déposé sur le coeur et donc forme une couche (ou écorce) déposée autour de ce coeur (ou support).

Le coeur des granulés peut également être considéré comme étant un support sur lequel vont se fixer les particules du principe actif.

Le coeur est constitué de particules solides et le principe actif supporté par ledit coeur est également sous forme solide.

La présente invention est donc basée sur la mise au point d'une nouvelle forme orale multiparticulaire.

Les granulés de l'invention présentent une couche de principe actif.

Les granulés de l'invention peuvent également comporter un ou plusieurs agents colorants. Ainsi, on choisit les colorants en fonction de leurs solubilités dans les solvants. Par exemple, on choisit un colorant pour sa solubilité dans l'éthanol et un autre pour sa solubilité dans l'eau. En effet, ces deux solvants sont les solvants habituellement utilisés pour extraire ou solubiliser les principes actifs.

La coloration obtenue permet alors de visualiser les ajouts malveillants dans une boisson, par exemple pour soumission chimique.

Les granulés de l'invention peuvent également comporter un ou plusieurs pigments métalliques. La présence de colorants et de pigments métalliques permet également de visualiser une solubilisation éventuelle après broyage de la forme pharmaceutique et par la suite une éventuelle ingestion. De même, en cas de mastication, on observe un phénomène identique.

Les colorants et les pigments métalliques peuvent être indifféremment placés dans les différentes couches des granulés de l'invention.

Avantageusement, on effectue un mélange intime du ou des colorants avec le principe actif et on utilise le pigment métallique dans la couche superficielle de la composition, c'est-à-dire celle qui est visible en surface.

Les granulés de l'invention comprennent également dans leur structure un ou plusieurs composés susceptibles de générer un dégagement gazeux lorsque la forme médicamenteuse se trouve hydratée.

Parmi les agents colorants des granulés de l'invention, on peut citer notamment les agents colorants solubles dans des solvants aqueux et les agents colorants solubles dans des solvants alcooliques.

Parmi les agents colorants solubles dans l'éthanol, on peut notamment citer les colorants suivants : rouge neutre, brillant bleu FDC...

Parmi les agents colorants solubles dans l'eau, on utilise les colorants classiques alimentaires. Les colorants mis en oeuvre dans le cadre de la présente invention sont notamment ceux listés dans la directive 95/45/CE du 26 juillet 1995 concernant les colorants pouvant être employés dans les denrées alimentaires (modifiée par la directive 2006/33/CE du 20 mars 2006). Ainsi, on peut notamment citer les colorants E 100 à E 180.

On peut également citer le colorant E131 (bleu patenté) soluble à la fois dans l'eau et dans l'éthanol.

Selon un mode de réalisation particulièrement préféré, les pigments métalliques des granulés de l'invention sont des pigments à base de dioxyde de titane présents à la surface dudit granulé.

De préférence, le coeur solide des granulés selon l'invention est un support insoluble. A titre de support insoluble, on utilise de préférence des polyols, des gommes, des dérivés de la silice, des dérivés de calcium ou de potassium, des composés minéraux tels que les phosphates dicalciques, des phosphates tricalciques et des carbonates de calcium, du saccharose, des dérivés de cellulose, notamment de la cellulose microcristalline, de l'éthylcellulose et de l'hydroxypropylméthylcellulose, de l'amidon, ou des mélanges de ceux-ci.

Les granulés de l'invention comprennent un coeur solide choisi de préférence parmi les supports insolubles dans des solvants aqueux ou alcooliques. Le choix de ces supports insolubles pour former le coeur solide des granulés de l'invention permet d'éviter une solubilisation totale du granulé en cas de broyage.

Le coeur solide des granulés peut également être constitué d'un mélange de composés, notamment d'un mélange de supports insolubles. Ainsi, on peut notamment citer le mélange formé de saccharose et d'amidon ou de composés minéraux dérivés de la silice ou du calcium.

Le coeur solide peut également être constitué de supports solubles parmi lesquels on peut citer certains grades solides de PEG (notamment PEG 4000 ou PEG 6000).

L'expression "dérivés de la silice" désigne la silice ainsi que les silices précipitées obtenues à partir de silicates alcalins, notamment Aerosil®, ou encore le talc, la bentonite ou le kaolin.

L'expression "dérivés de calcium" désigne des excipients cristallins dérivés de l'hydroxyde de calcium, des produits insolubles dans l'eau utilisés en médecine comme diluants, ou des agents de charges et également abrasifs.

L'expression "dérivés de potassium" désigne notamment le bicarbonate de potassium et le chlorure de potassium.

Parmi les supports insolubles formant le coeur des granulés de l'invention, on peut également citer les dérivés du magnésium (notamment carbonates ou oxydes).

De préférence, on utilise des sphères de sucre à titre de support solide formant le coeur des granulés de l'invention. Ces sphères sont constituées d'un mélange de saccharose et d'amidon.

Selon un mode préféré, les granulés susmentionnés comprennent également un liant. Le rôle du liant est de lier entre elles les particules, c'est-à-dire de parfaire la cohésion du granulé. Ainsi, les liants permettent d'assurer une bonne cohésion du principe actif et du coeur dans les granulés.

Ainsi, les liants se trouvent, comme le principe actif, déposés autour du coeur des granulés.

Comme liants, on peut citer la plupart des excipients hydrophiles qui donnent des solutions visqueuses : gommes arabique et adragante, méthylcellulose et carboxyméthylcellulose, gélatine, amidons, maltodextrines, PEG 4000 et 6000 en solution alcoolique, polyvidone en solution aqueuse ou alcoolique, et aussi des solutions de saccharose, de glucose ou de sorbitol.

Les liants des granulés de l'invention sont de préférence choisis dans le groupe constitué de l'amidon, du saccharose, de la gomme arabique, de la polyvinylpyrrolidone (PVP ou polyvidone), de l'hydroxypropylméthylcellulose (HPMC), de la gomme laque, de l'hydroxypropylcellulose (HPC), de la cellulose, des polyols ou des alginates, des glycérides polyglycolysés (Gelucire®) ou des macrogolglycérides, notamment macrogolglycérides de stéaroyle, également dérivés acryliques, ainsi que des mélanges de ceux-ci.

Parmi les polyols, on peut citer notamment le mannitol, le sorbitol, le maltitol ou le xylitol.

Selon un mode de réalisation particulier, les liants sont de préférence choisis dans le groupe constitué de la polyvinylpyrrolidone, de la gomme laque, des polyols ou des alginates, des glycérides polyglycolysés (Gelucire) ou des macrogolglycérides, notamment macrogolglycérides de stéaroyle, ainsi que des mélanges de ceux-ci.

On peut également utiliser un liant choisi dans les groupes cités ci-dessus pour des propriétés particulières ; par exemple il peut être utile d'utiliser comme liant des excipients pH-dépendants tels que EUDRAGlT® L100 ou de la gomme laque. On peut également choisir d'utiliser préférentiellement des glycérides polyglycolysés (Gelucire®) pour leur caractère hydrophobe.

Selon un mode de réalisation préféré, les granulés de l'invention comprennent en outre un ou plusieurs agents d'amertume.

De préférence, ledit agent d'amertume est choisi dans le groupe constitué du benzoate de dénatonium, des extraits de gentianes, de la quinine, de la caféine, de la brucine, de la quassine, du propyl thiouracile (PROP), du phénylthiocarbamide (PTC), des composés astringents tels que les tannins, des arômes de pamplemousse et des arômes de cacao amer.

La présence dudit agent d'amertume (ou promoteur d'amertume)(ex : Bitrex® - benzoate de dénatonium) en mélange intime avec le principe actif rend difficile voire impossible une absorption par mastication accidentelle ou même après extraction et/ou solubilisation. En effet, ledit agent d'amertume se trouve alors solubilisé en même temps que le principe actif, la séparation différentielle étant très difficile.

L'utilisation d'un tel composé amer permet de prévenir les administrations volontaires ou dissimulées sous forme de "cocktails" dans des mélanges eau/alcool (glaçons/vodka etc...).

Selon un mode de réalisation particulièrement préféré, le coeur solide des granulés de l'invention représente de 15% à 30%, et de préférence de 20% à 25%, en poids par rapport au poids total du granulé.

Parmi les diluants préférés selon l'invention, on peut citer les dérivés de silice, et notamment les dérivés du magnésium aluminométasilicate.

Parmi les dérivés du magnésium aluminométasilicate, on peut notamment citer le produit Neusilin® qui présente un intérêt relativement au caractère très hygroscopique du GHB.

De préférence, le composé susceptible de générer un dégagement gazeux est choisi dans le groupe constitué des carbonates et bicarbonates, et est notamment choisi dans le groupe constitué du bicarbonate de sodium, du carbonate de sodium, du carbonate de glycine de sodium, du bicarbonate de potassium, du carbonate de magnésium et du carbonate de calcium.

Selon un mode de réalisation préféré, le granulé selon l'invention comprend du bicarbonate de sodium à titre de composé susceptible de générer un dégagement gazeux.

De façon préférée, le principe actif des granulés de l'invention est le sel de sodium de l'acide gamma-hydroxybutyrique.

Selon un mode de réalisation, les granulés selon la présente invention peuvent comprendre une membrane constituée d'excipients d'enrobage pour libération immédiate. De tels granulés sont désignés par la suite comme des granulés à libération immédiate.

Selon un autre mode de réalisation, les granulés selon la présente invention peuvent comprendre une membrane constituée d'excipients d'enrobage pour libération étalée ou progressive. De tels granulés sont désignés par la suite comme des granulés à libération étalée progressive.

La présente invention concerne également une composition pharmaceutique comprenant un mélange de granulés tels que définis ci-dessus, dans laquelle ledit mélange est constitué de deux groupes de granulés (A) et (B), les granulés (A) et les granulés (B) présentant des cinétiques différentes de libération du principe actif.

Cette forme galénique spécifique consiste en la combinaison de deux types de granules présentant des cinétiques différentes. Une telle composition permet d'obtenir des résultats *in vivo* cohérents avec ce qui est observé *in vitro.* Par ailleurs, le fait d'utiliser une forme multi-particulaire permet de diminuer la forte variabilité interindividuelle observée pour ce principe actif, comparativement aux formes monolithiques.

La présente invention concerne également une composition pharmaceutique comprenant des granulés à libération immédiate et des granulés à libération étalée/progressive.

Selon un mode de réalisation préféré, la présente invention concerne une composition pharmaceutique comprenant des granulés à libération immédiate, à raison de 5% à 50% en poids, et des granulés à libération étalée/progressive, à raison de 50% à 95% en poids. Préférentiellement, ladite composition pharmaceutique comprend 25% en poids de granulés à libération immédiate et 75% en poids de granulés à libération étalée/progressive.

La présente invention concerne également un granulé tel que défini ci-dessus, ou une composition pharmaceutique telle que définie ci-dessus, pour son utilisation pour le traitement de la catalepsie chez les patients narcoleptiques, ou pour son utilisation pour le sevrage de l'alcoolisme.

La présente invention concerne également un granulé tel que défini ci-dessus, ou une composition pharmaceutique telle que définie ci-dessus, pour son utilisation pour la prévention et/ou le traitement de la fibromyalgie, pour la prévention des rechutes alcooliques et le maintien de l'abstinence, ou encore pour le traitement de l'anxiété chez l'alcoolique.

La présente invention concerne également un procédé de préparation d'un granulé tel que défini ci-dessus, caractérisé en ce qu'il comprend une étape d'application par poudrage du principe actif sur le coeur solide.

Selon un mode de réalisation préféré du procédé de l'invention, le principe actif est mélangé avec les composés susceptibles de générer un dégagement gazeux, les diluants, les éventuels agents colorants et les éventuels pigments métalliques avant l'étape d'application par poudrage sur le coeur solide.

Le procédé de l'invention peut également comprendre, après l'étape de poudrage, une étape d'enrobage du granulé, notamment en déposant par pelliculage l'agent enrobant sous forme de film sur le granulé, suivie, le cas échéant d'une étape de mélange avec un lubrifiant et/ou un arôme et/ou un édulcorant et/ou un colorant ou pigment métallique.

La structure des granulés de l'invention est liée à la mise en oeuvre de ce procédé particulier qui permet l'obtention de granulés de structure coeur-écorce.

En effectuant des essais comparatifs de préparation de granulés par un procédé de granulation directe avec différents excipients habituellement utilisés en granulation, il a été constaté que les résultats obtenus concernant le granulé lui-même sont satisfaisants concernant l'aspect, la friabilité et la dissolution. Cependant, les granulés obtenus par un tel procédé présentent une surface spécifique très élevée nécessitant des quantités importantes de polymères d'enrobage selon les techniques conventionnellement utilisées.

Ainsi, les granulés de la présente invention sont caractérisés en ce qu'ils présentent une surface spécifique abaissée. Par ailleurs, d'aspect, ils sont relativement lisses et présentent une forme plutôt régulière.

L'étape de poudrage susmentionnée du procédé pour la préparation des granulés de l'invention peut également comprendre une étape de pulvérisation d'une solution alcoolique ou hydroalcoolique ou aqueuse d'un liant.

Cette étape de pulvérisation et l'étape de poudrage sont de préférence effectuées de façon simultanée ou alternée.

De préférence, l'étape de poudrage susmentionnée est effectuée de façon concomitante avec une étape de pulvérisation d'un liant sous forme de solution.

La combinaison de ces étapes permet d'assurer une bonne cohésion du principe actif sur le coeur des granulés.

Une mise en oeuvre avantageuse du procédé de l'invention consiste ainsi à appliquer le principe actif sous forme de poudre sur le support particulaire susmentionné (ou coeur des granulés) en alternant des séquences de pulvérisation du liant sous forme de solution.

Le procédé de l'invention peut également comprendre, après l'étape précédente, une ou plusieurs étapes d'enrobage du granulé, notamment en déposant par pelliculage le ou les agents enrobants sous forme de films sur le granulé.

La faible surface spécifique des granulés de l'invention permet ainsi, en cas d'enrobage, de réduire la quantité utilisée d'agent d'enrobage et donc de moins diluer le principe actif dans lesdits granulés enrobés.

Un mode de réalisation préféré du procédé de l'invention consiste en un procédé comprenant, après l'étape d'enrobage, une étape de mélange avec un lubrifiant et/ou un arôme et/ou un édulcorant, ceux-ci pouvant être eux-mêmes préparés sous forme de granulés afin d'être finalement mélangés aux granulés actifs.

Toutefois les lubrifiants, arômes et édulcorants peuvent être également ajoutés avant l'étape de poudrage susmentionnée.

### EXEMPLES

### I - Protocoles de préparation des granulés de l'invention

Le principe actif est mélangé pendant quinze minutes avec le composé susceptible de générer un dégagement gazeux et le diluant dans un mélangeur par retournement. Le mélange obtenu est ensuite broyé sur un broyeur type Forplex F1, de manière à obtenir une granulométrie adéquate. Le mélange actif est alors déposé sur les supports neutres (coeur solide). Les granules sont placés dans une turbine conventionnelle, le mélange est déposé par poudrage en alternant des phases de poudrage et des phases de pulvérisation d'une solution liante.

A l'issue de cette étape de poudrage, on effectue une phase de séchage de manière a éliminer les solvants utilisés lors de l'étape précédente

On procède ensuite a l'étape d'enrobage. Pour cela, les granules issus de l'étape précédente sont placés dans un lit d'air fluidisé type GPCG 30 et la solution d'enrobage (pour la membrane) est alors pulvérisée sur la masse de granules en fluidisation.

Après une dernière étape de séchage, on procède à la phase de lubrification, l'étape ultime du procédé, qui consiste au conditionnement en sticks, sachets, ampoules ou flacons.

### II - Exemples de formulations selon l'invention

### Exemple 1 (libération immédiate)

| **Formule N° 1** | |
|---|---|
| Matières | % |
| GHB (sel hydraté) | 57,60 |
| Bicarbonate de sodium | 8,64 |
| Neusilin® UFL2 (diluant) | 5,18 |
| Gomme laque décirée blanchie (liant) | 4,60 |
| Sphères de sucre (coeur solide) | 20,57 |
| Sepifilm LP014 (membrane protectrice contre l'humidité | 2,90 |
| Talc | 0,50 |
| Masse théorique | 100,0 |

### Exemple 2 (libération modifiée)

| **Formule N° 2** | |
|---|---|
| Matières | % |
| GHB (sel hydraté) | 56,49 |
| Bicarbonate de sodium | 8,47 |
| Neusilin® UFL2 (diluant) | 5,09 |
| Gomme laque décirée blanchie (liant) | 7,12 |
| Sphères de sucre (coeur solide) | 20,08 |
| Sépifilm LP014 (membrane protectrice contre l'humidité) | 0,76 |
| Pharmacoat 603 (membrane de diffusion) | 1,15 |
| Talc | 0,85 |
| Masse théorique | 100,0 |

### Exemple 3 (Mélange 50/50)

| **Formule N° 3** | |
|---|---|
| Matières | % |
| GHB (sel hydraté) | 57,04 |
| Bicarbonate de sodium | 8,56 |
| Neusilin® UFL2 (diluant) | 5,13 |
| Gomme laque décirée blanchie (liant) | 5,87 |
| Sphères de sucre (coeur solide) | 20,32 |
| Sépifilm LP014 (membrane protectrice contre l'humidité | 1,82 |
| Pharmacoat 603 (membrane de diffusion) | 0,58 |
| Talc | 0,68 |
| Masse théorique | 100,0 |

### Exemple 4 (libération immédiate)

| **Formule N° 4** | |
|---|---|
| Matières | % |
| GHB (sel hydraté) | 56,47 |
| Bicarbonate de sodium | 8,47 |
| Neusilin® UFL2 (diluant) | 5,08 |
| Gomme laque décirée blanchie (liant) | 6,56 |
| Sphères de sucre (coeur solide) | 19,88 |
| Enrobage protecteur | 3,04 |
| Talc | 0,50 |
| Masse théorique | 100,0 |

### Exemple 5 (libération modifiée)

| **Formule N° 5** | |
|---|---|
| Matières | % |
| GHB (sel hydraté) | 54,75 |
| Bicarbonate de sodium | 8,22 |
| Neusilin® UFL2 (diluant) | 4,91 |
| Gomme laque décirée blanchie (liant) | 9,79 |
| Sphères de sucre (coeur solide) | 19,27 |
| Enrobage protecteur | 1,89 |
| Talc | 1,18 |
| Masse théorique | 100,0 |

### Exemple 6 (mélange de granulés 25-75)

| **Formule N° 6** | |
|---|---|
| Matières | % |
| GHB (sel hydraté) | 55,17 |
| Bicarbonate de sodium | 8,28 |
| Neusilin® UFL2 (diluant) | 4,95 |
| Gomme laque décirée blanchie (liant) | 9,00 |
| Sphères de sucre (coeur solide) | 19,42 |
| Enrobage protecteur | 2,16 |
| Talc | 1,02 |
| Masse théorique | 100,0 |

### III - Etudes de stabilité et de dissolution

### Méthode analytique

Les formulations présentées ont été testées analytiquement et leur dissolution a été étudiée afin d'anticiper *in vitro* le comportement *in vivo* des différentes formulations. La méthode retenue est la suivante :
Les essais sont soumis à une agitation constante de 100 tpm dans 900 ml d'acide chlorhydrique 0,1N, à 37°C dans un appareil de dissolution équipé de pales (USP apparatus 2). Des prélèvements sont réalisés grâce à un collecteur d'échantillons, à 5, 10, 15 et 30 min, puis analysés en HPLC.

Les résultats des tests de dissolution effectués sur les différentes formulations sont présentés ci-dessous.

Le comportement dans le temps des formulations a également été étudié : des sticks ainsi que des ampoules ont été réalisés a partir des formulations présentées et après répartition en enceintes climatiques, une étude de stabilité a été conduite selon les normes ICH en vigueur.

Les résultats de ces études sont présentés ci-dessous.

On peut constater pour les deux conditionnements présentés l'absence de modification significative des paramètres évalués. Les formulations démontrent une stabilité parfaitement acceptable au regard des normes ICH.

### III.1. Résultats avec la formule N°1 selon l'exemple 1

**Résultats en ampoules de 1,75 g à 25°C/60% HR s**

| Points test | T0 | T6 mois | T12 mois | T18 mois |
|---|---|---|---|---|
| Caractéristiques des granulés | | | | |
| Couleur | Blanchâtre | Blanchâtre | Blanchâtre | Blanchâtre |
| Forme | Sphérique | Sphérique | Sphérique | Sphérique |
| Aspect | Homogène | Homogène | Homogène | Homogène |

| Dosage | | | | |
|---|---|---|---|---|
| Teneur (g/ampoule) | 1,753 | 1,753 | 1,736 | 1,758 |
| % / théorie | 100,2 | 100,2 | 99,2 | 100,5 |

| Dosage des impuretés | | | | |
|---|---|---|---|---|
| Somme totale (%) | < LD (limite de détection) | < LQ (limite de quantification) | < LQ | < LD |

| Test de dissolution | | | | |
|---|---|---|---|---|
| Temps (min) | % dissous | | | |
| 5 | --- | 88,7 | 90,1 | 87,3 |
| 10 | --- | 92,5 | 97,9 | 94,9 |
| 15 | 93,5 | 94,6 | 98,6 | 98,2 |
| 30 | 97,8 | 97,1 | 99,9 | 99,6 |

Les résultats de cette étude de stabilité sont satisfaisants au regard des standards ICH.

**Résultats en ampoules de 1,75 g à 40°C/75% HR**

| Points test | T0 | T2 mois | T3 mois | T6 mois |
|---|---|---|---|---|
| Caractéristiques des granules | | | | |
| Couleur | Blanchâtre | Blanchâtre | Blanchâtre | Blanchâtre |
| Forme | Sphérique | Sphérique | Sphérique | Sphérique |
| Aspect | Homogène | Homogène | Homogène | Homogène |

| Dosage | | | | |
|---|---|---|---|---|
| Teneur (g/ampoule) | 1,753 | 1,765 | 1,753 | 1,723 |
| % / théorie | 100,2 | 100,9 | 100,2 | 98,5 |

| Dosage des impuretés | | | | |
|---|---|---|---|---|
| Somme totale (%) | < LD | < LQ | < LQ | < LD |

| Test de dissolution | | | | |
|---|---|---|---|---|
| Temps (min) | % dissous | | | |
| 5 | --- | --- | --- | 77,0 |
| 10 | --- | --- | --- | 88,3 |
| 15 | 93,5 | 95,9 | 90,6 | 91,8 |
| 30 | 97,8 | 99,4 | 93,3 | 96,8 |

Les résultats de cette étude de stabilité sont satisfaisants au regard des standards ICH.

### III.2. Résultats avec la formule N°3 selon l'exemple 3

**Résultats en ampoules de 1,50 g à 25°C/60% HR**

| Points test | T0 | T5 mois | T9 mois | T12 mois |
|---|---|---|---|---|
| Caractéristiques des granules | | | | |
| Couleur | Blanchâtre | Blanchâtre | Blanchâtre | Blanchâtre |
| Forme | Sphérique | Sphérique | Sphérique | Sphérique |
| Aspect | Homogène | Homogène | Homogène | Homogène |

| Dosage | | | | |
|---|---|---|---|---|
| Teneur (g/ampoule) | 1,502 | 1,485 | 1,496 | 1,515 |
| % / théorie | 100,1 | 99,0 | 99,7 | 101,0 |

| **Dosage des impuretés** | | | | |
|---|---|---|---|---|
| Somme totale (%) | < LD | < LD | < LD | < LD |
| **Solvants résiduels** | | | | |
| Ethanol (ppm) | 414 | < LD | < LQ | < LQ |

| **Test de dissolution** | | | | |
|---|---|---|---|---|
| Temps (min) | % dissous | | | |
| 5 | 50,8 | 50,4 | 50,2 | 49,4 |
| 15 | 55,4 | 56,2 | 56,0 | 54,4 |
| 60 | 72,5 | 72,8 | 73,4 | 72,0 |
| 120 | 85,3 | 87,4 | 88,4 | 87,0 |
| 180 | 92,1 | 94,6 | 96,1 | 94,5 |

Les résultats de cette étude de stabilité sont satisfaisants au regard des standards ICH.

**Résultats en ampoules de 1,50 g à 40°C/75% HR**

| Points test | T0 | T1 mois | T3 mois | T7 mois |
|---|---|---|---|---|
| **Caractéristiques des granules** | | | | |
| Couleur | Blanchâtre | Blanchâtre | Blanchâtre | Blanchâtre |
| Forme | Sphérique | Sphérique | Sphérique | Sphérique |
| Aspect | Homogène | Homogène | Homogène | Homogène |

| **Dosage** | | | | |
|---|---|---|---|---|
| Teneur (g/ampoule) | 1,502 | 1,485 | 1,506 | 1,500 |
| % / théorie | 100,1 | 99,0 | 100,3 | 100,0 |

| **Dosage des impuretés** | | | | |
|---|---|---|---|---|
| Somme totale (%) | < LD | < LQ | < LQ | < LD |

| **Solvants résiduels** | | | | |
|---|---|---|---|---|
| Ethanol (ppm) | 414 | 205 | < LQ | < LQ |

| **Test de dissolution** | | | | |
|---|---|---|---|---|
| Temps (min) | % dissous | | | |
| 5 | 50,8 | 49,2 | 49,0 | 49,3 |
| 15 | 55,4 | 55,5 | 56,5 | 55,2 |
| 60 | 72,5 | 73,0 | 72,3 | 71,0 |
| 120 | 85,3 | 86,1 | 86,8 | 86,8 |
| 180 | 92,1 | 94,5 | 93,8 | 95,7 |

Les résultats de cette étude de stabilité sont satisfaisants au regard des standards ICH.

### IV - Résultats de dissolution in vitro

**- Libération immédiate**

| Temps (h) | % dissous | |
|---|---|---|
| | SMO.IR A001 (exemple 1) | SMO.IR A002 (exemple 4) |
| 0 | 0,0 | 0,0 |
| 0,0833 | 88,7 | 79,6 |
| 0,1667 | 92,5 | 86,0 |
| 0,25 | 94,6 | 89,2 |
| 0,5 | 97,1 | 95,1 |

### - Libération étalée

| Temps (h) | % dissous | |
|---|---|---|
| | SMO.SR A001 (exemple 2) | SMO.SR A002 (exemple 5) |
| 0 | 0,0 | 0,0 |
| 0,25 | 9,6 | 10,5 |
| 0,5 | 20,6 | 22,4 |
| 1 | 41,5 | 45,8 |
| 2 | 72,0 | 75,6 |
| 3 | 87,0 | 90,7 |
| 4 | 94,8 | |

### - Mélange 50/50

| Temps (h) | % dissous |
|---|---|
| | SMO.MR A001 (exemple 3) |
| 0 | 0,0 |
| 0,0833 | 50,8 |
| 0,25 | 55,4 |
| 0,5 | 59,0 |
| 1 | 72,5 |
| 2 | 85,3 |
| 3 | 92,1 |

Une étude de relation *in vitro*/*in vivo* a été conduite sur les formulations tests et références. Les formulations tests ont été développées dans le cadre de la présente invention et se présentent sous forme de microgranules telles que décrites précédemment.

Deux références de formulations orales sont sur le marché : ALCOVER® et XYREM®. Elles sont sous forme liquide, en flacon. Les bioéquivalences des microgranules, versus solutions ont été étudiées dans des études cliniques aux dosages suivants : 1750 et 2250 mg.

Le premier objectif de cette étude est de décrire la cinétique d'absorption intestinale de l'acide gamma hydroxybutyrique chez l'Humain, après son administration orale sous forme de solution ou sous forme de microgranules, afin de savoir si son profil et sa vitesse de dissolution *in vitro* à partir des microgranules influent sur le profil des concentrations plasmatiques de l'acide gamma hydroxybutyrique ainsi que sur les paramètres pharmacocinétiques.

Le deuxième objectif est d'analyser la cinétique d'absorption in vivo des microgranules et de la comparer à celle des formes orales solutions sur le marché

L'exposition à l'acide gamma hydroxybutyrique (AUCinf) n'est pas dose-dépendante pour les doses testées (1750-2250mg), l'absorption et l'exposition présentent une bonne proportionnalité par rapport aux doses, ce qui permet de déterminer et de comparer les cinétiques d'absorption aux deux dosages.

Les cinétiques d'absorptions orales exprimées en % de dose absorbés cumulés puis en vitesses d'absorption en fonction du temps ont été calculées par une méthode de déconvolution pharmacocinétique compartimentale (Wagner JG, Nelson E.) pour les solutions d'ALCOVER® et de XYREM® et pour les microgranules, administrés à des volontaires sains aux doses unitaires de 1750 mg and 2250 mg (dans deux études séparées).

Dans les cas des solutions et des microgranules, les cinétiques d'absorption systémique cumulatives d'acide gamma hydroxybutyrique sont ensuite analysées pour caractériser mathématiquement leurs profils en calculant les constantes des équations qui décrivent l'absorption systémique d'acide gamma hydroxybutyrique (courbes cumulatives et vitesses en fonction du temps). Les modèles d'absorption ainsi que les équations sont reportés.

Pour les solutions testées à 1750 et 2250 mg, les cinétiques d'absorption sont très rapides; la durée totale du mécanisme d'absorption est inférieure à 1 heure.

Le temps nécessaire à 50% d'absorption de la dose (T50%) est très court de 0,21 à 0,28 heure, les vitesses maximum d'absorption (314 à 362 %/h) sont observées tôt, à 0,33 heure. Tous les mécanismes d'absorption suivent des cinétiques de 1^{er} ordre ; les constantes d'absorption sont très élevées: 4,5 à 9,3 H-1, confirmant une absorption orale très rapide des solutions d'acide gamma-hydroxybutyrique.

Pour les microgranules, les cinétiques d'absorption cumulatives, les vitesses d'absorption en fonction du temps ainsi que les paramètres sont superposables à ceux obtenus après administration des deux solutions, quelque soit le dosage ; les constantes des équations décrivant les cinétiques d'absorption sont similaires.

Les processus de dissolution *in vivo* des microgranules sont si rapides qu'ils n'influent pas sur les profils et les paramètres cinétiques d' absorption de l'acide gamma hydroxybutyrique.

Les cinétiques de dissolution *in vitro* de l'acide gamma hydroxybutyrique à partir des microgranules sont très rapides, plus de 85% de la dose est dissoute en moins de 5 minutes dans toutes les conditions de pH physiologique testées (pH 1,2 à 6,8) quelque soient les conditions *in vitro.*

La très grande vitesse de dissolution de l'acide gamma hydroxybutyrique à partir des microgranules explique l'absence de différence de vitesse d'absorption *in vivo* entre les solutions et les microgranules.

La dissolution des microgranules n'affecte pas la cinétique d'absorption de l'acide gamma hydroxybutyrique, qui est superposable à celle des solutions disponibles sur le marché.

Les paramètres d'absorption ont été étudiés selon la méthode déconvolution pharmacocinétique compartimentale de Wagner JG, Nelson E. [J. Pharm. Sci. 1968 ; 53(11) :1392-1403]. Cette méthode pharmacocinétique basée sur le calcul des aires sous courbes des concentrations plasmatiques moyennes en fonction du temps et de la pente d'élimination terminale des courbes moyennes permet de calculer à chaque temps de la cinétique plasmatique le % de dose entré dans la circulation systémique en relatif à la quantité totale entrée à la fin de l'absorption ; cette méthode aboutit donc à caractériser de 0 à 100 % le profil de l'entrée systémique des formulations testées. La condition d'application de la méthode à été vérifiée : la pente terminale moyenne d'élimination calculée par régression semi logarithmique sur la partie linéaire des courbes plasmatiques de 1,5 à 4 heures des microgranules est similaire à celle des solutions et correspond à la demi-vie plasmatique (T1/2) très courte de l'acide gamma hydroxybutyrique : tableau ci-dessous

| ALCOVER1750 | | XYREM1750 | MICROG1750 | ALCOVER2250 | MICROG2250 |
|---|---|---|---|---|---|
| Pente (H-1) | 1,06 | 1,11 | 0,85 | 1,03 | 1,02 |
| T1/2 (H) | 0,65 | 0,62 | 0,81 | 0,68 | 0,68 |

Les courbes et tableaux suivants présentent les pourcentages d'absorption ainsi que les ratios d'absorption des formes test et références.

### Dosage 1750 mg : Référence Alcover

| IN VIVO HUMAIN - ETUDE PK SMO212/09/01 | | | **ETUDE PK SMO212/09/01 ABSORPTION ORALE MOYENNE %DOSE MICRO-GR. IR A001 Lot 4SMOI001 et ALCOVER 1750 mg** |
|---|---|---|---|
| RESULTATS D'ABSORPTION : % DOSE | | | 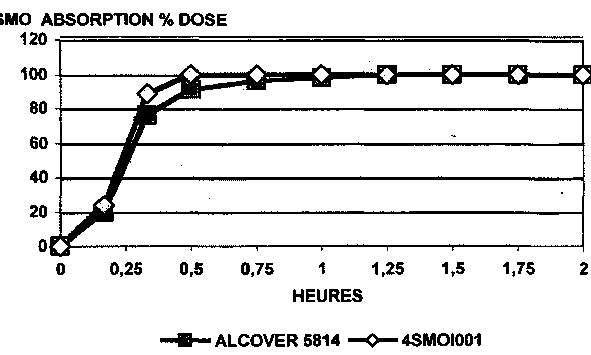 |
| TEMPS (HEURES) | SOLUTION ALCOVER 5814 | MICROG-IR 4SMOI001 | |
| 0 | 0 | 0 | |
| 0,17 | 19,18 | 23,62 | |
| 0,33 | 76,25 | 88,37 | |
| 0,50 | 91,13 | 100,00 | |
| 0,75 | 95,97 | 100,00 | |
| 1,00 | 97,68 | 100,00 | |
| 1,25 | 100,00 | 100,00 | |
| 1,50 | 100,00 | 100,00 | |
| 1,75 | 100,00 | 100,00 | |
| 2,00 | 100,00 | 100,00 | |
| 2,50 | | | |

| IN VIVO HUMAIN - ETUDE PK SMO212/09/01 | | | **ETUDE PK SMO212/09/01 VITESSE MOYENNE D'ABSORPTION ORALE %Dose/H MICRO-GR. IR A001 Lot 4SMOI001 et ALCOVER 1750 mg** |
|---|---|---|---|
| RESULTATS VITESSE D'ABSORPTION: % DOSE/H | | | 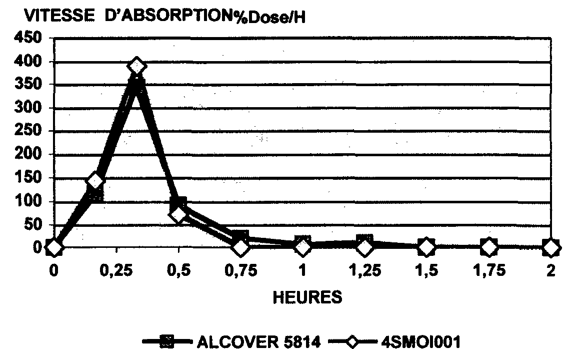 |
| TEMPS (HEURES) | SOLUTION ALCOVER 5814 | MICROG-IR 4SMO1001 | |
| 0 | 0 | 0 | |
| 0,17 | 115,07 | 141,67 | |
| 0,33 | 342,57 | 388,66 | |
| 0,50 | 89,26 | 69,78 | |
| 0,75 | 19,36 | 0,00 | |
| 1,00 | 6,81 | 0,00 | |
| 1,25 | 9,30 | 0,00 | |
| 1,50 | 0,00 | 0,00 | |
| 1,75 | 0,00 | 0,00 | |
| 2,00 | 0,00 | 0,00 | |
| 2,50 | | | |

Les cinétiques d'absorption de l'acide gamma hydroxybutyrique (% Dose et vitesse) présentent les mêmes profils et sont superposables.

Les microgranules IR n'induisent pas de retard ni de modification du processus d' absorption de l'acide gamma hydroxybutyrique par rapport à la solution référence Alcover. La dissolution *in vivo* dans l'estomac est très rapide et totale.

### Dosage 1750 mg: Référence Xvrem:

| IN VIVO HUMAIN- ETUDE PK SMO212/09/01 | | | **ETUDE PK SMO212/09/01 ABSORPTION ORALE MOYENNE %DOSE MICRO-GR. IR A001 Lot 4SMOI001 et XYREM 1750 mg** |
|---|---|---|---|
| RESULTATS D'ABSORPTION : % DOSE | | | 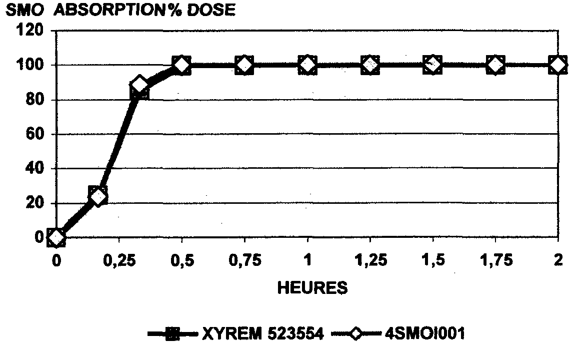 |
| TEMPS (HEURES) | SOLUTION XYREM 523554 | MICROG-IR 4SMOI001 | |
| 0 | 0 | 0 | |
| 0,17 | 24,61 | 23,62 | |
| 0,33 | 84,87 | 88,37 | |
| 0,50 | 99,67 | 100,00 | |
| 0,75 | 99,67 | 100,00 | |
| 1,00 | 99,67 | 100,00 | |
| 1,25 | 99,67 | 100,00 | |
| 1,50 | 99,67 | 100,00 | |
| 1,75 | 100,00 | 100,00 | |
| 2,00 | 100,00 | 100,00 | |
| 2,50 | | | |

| IN VIVO HUMAIN-ETUDE PK SMO212/09/01 | | | **ETUDE PK SMO212/09/01 VITESSE MOYENNE D'ABSORPTION ORALE %Dose/H MICRO-GR. IR A001 Lot 4SMOI001 et XYREM 1750 mg** |
|---|---|---|---|
| RESULTAT VITESSE D'ABSORPTION : % DOSE/H | | | 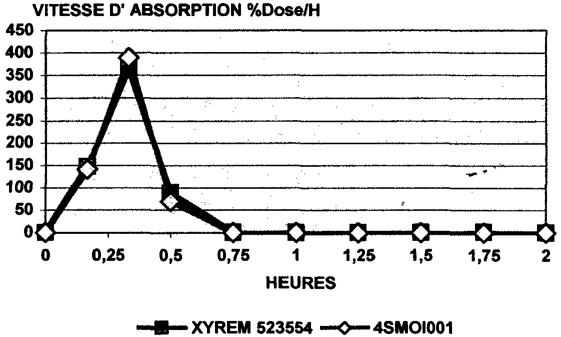 |
| TEMPS (HEURES) | SOLUTION XYREM 523554 | MICROG-IR 4SMOI001 | |
| 0 | 0 | 0 | |
| 0,17 | 147,64 | 141,79 | |
| 0,33 | 361,67 | 388,98 | |
| 0,50 | 88,80 | 69,34 | |
| 0,75 | 0,00 | 0,00 | |
| 1,00 | 0,00 | 0,00 | |
| 1,25 | 0,00 | 0,00 | |
| 1,50 | 0,00 | 0,00 | |
| 1,75 | 1,32 | 0,00 | |
| 2,00 | 0,00 | 0,00 | |
| 2,50 | | | |

Les cinétiques d'absorption de l'acide gamma hydroxybutyrique (% Dose et Vitesse) présentent les mêmes profils et sont superposables.

Les microgranules IR n'induisent pas de retard ni de modification du processus d' absorption de l'acide gamma hydroxybutyrique par rapport à la solution référence Xyrem. La dissolution *in vivo* dans l'estomac est très rapide et totale.

### Dosage 1750 mg :Formulation microgranules versus Alcover et Xvrem :

| ETUDE PK SMO212/09/01 RAPPORT MOYEN D'ABSORPTION ORALE | | **ETUDE PK SMO212/09/01 RAPPORT MOYEN D'ABSORPTION ORALE MICRO-GR. IR A001 Lot 4SMOI001 VERSUS ALCOVER 1750 mg** |
|---|---|---|
| MICRO-GR. IR A001 Lot 4SMOI001 VERSUS ALCOVER 1750mg | | 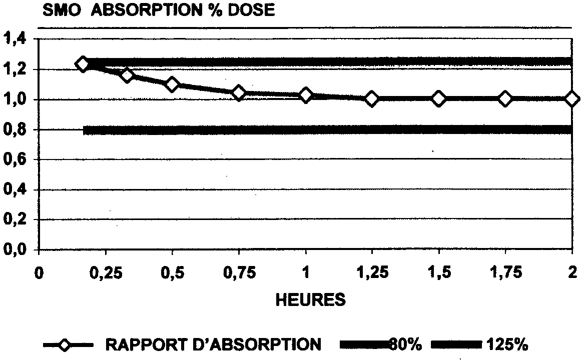 |
| HEURES | MICROG-IR RAPPORT D'ABSORPTION | |
| 0 | | |
| 0,17 | 1,23 | |
| 0,33 | 1,16 | |
| 0,50 | 1,10 | |
| 0,75 | 1,04 | |
| 1,00 | 1,02 | |
| 1,25 | 1,00 | |
| 1,50 | 1,00 | |
| 1,75 | 1,00 | |
| 2,00 | 1,00 | |

Le rapport des profils d'absorption des microgranules IR 1750 mg et de la solution Alcover® est inclus entre les valeurs 0,8 et 1,2

| ETUDE PK SMO212/09/01 RAPPORT MOYEN D'ABSORPTION ORALE | | | **ETUDE PK SMO212/09/01 RAPPORT MOYEN D'ABSORPTION ORALE MICRO-GR. IR A001 Batch 4SMOI001 VERSUS XYREM 1750** |
|---|---|---|---|
| MICRO-GR. IR A001 Batch 4SMOI001 VERSUS XYREM 1750mg | | | 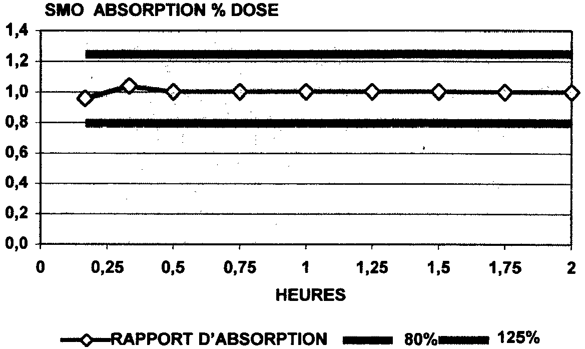 |
| HEURES | MICROG-IR RAPPORT D'ABSORPTION | | |
| 0 | | | |
| 0,17 | 0,96 | | |
| 0,33 | 1,04 | | |
| 0,50 | 1,00 | | |
| 0,75 | 1,00 | | |
| 1,00 | 1,00 | | |
| 1,25 | 1,00 | | |
| 1,50 | 1,00 | | |
| 1,75 | 1,00 | | |
| 2,00 | 1,00 | | |

Le rapport R des profils d'absorption des microgranules IR 1750 mg et de la solution XYREM ® est inclus entre les valeurs 0,8 et 1,2.

### Dosage 2250 ma : Référence Alcover

| IN VIVO HUMAIN-ETUDE PK SMO032/10/11 | | | **PK STUDY SMO032/10/01 ABSORPTION ORALE MOYENNE %DOSE MICRO-GR. IR B001 Lot 4SMOI002 et ALCOVER 2250 mg** |
|---|---|---|---|
| RESULTATS D'ABSORPTION : % DOSE | | | 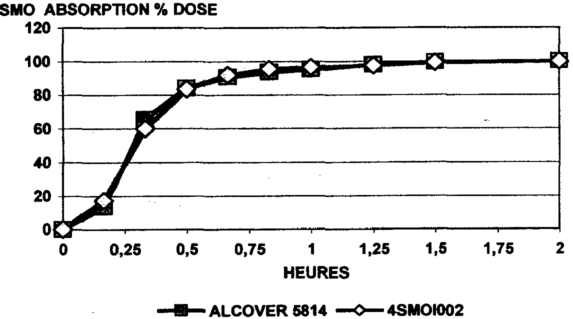 |
| TEMPS (HEURES) | SOLUTION ALCOVER 5814 | MICROG-IR 4SMOI002 | |
| 0 | 0 | 0 | |
| 0,17 | 13,09 | 17,05 | |
| 0,33 | 65,56 | 59,99 | |
| 0,50 | 83,90 | 83,52 | |
| 0,67 | 90,33 | 91,79 | |
| 0,83 | 93,59 | 95,41 | |
| 1,00 | 95,18 | 96,46 | |
| 1,25 | 97,99 | 97,44 | |
| 1,50 | 99,63 | 99,22 | |
| 2,00 | 100,00 | 100,00 | |
| 2,50 | 100,00 | 100,00 | |

| IN VIVO HUMAIN- ETUDE PK SMO212/09/01 | | | **PK STUDY SMO032/10/01 VITESSE MOYENNE D'ABSORPTION ORALE %Dose/H MICRO-GR. IR B001 Lot 4SMOI002 et ALCOVER 2250 mg** |
|---|---|---|---|
| VITESSE D'ABSORPTION : % DOSE/H | | | 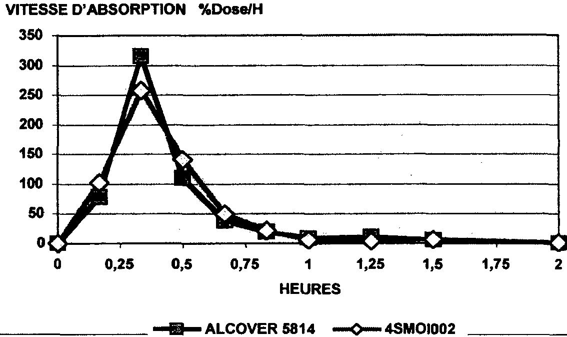 |
| TEMPS (HEURES) | SOLUTION ALCOVER 5814 | MICROG-IR 4SMOI001 | |
| 0 | 0 | 0 | |
| 0,17 | 115,07 | 141,67 | |
| 0,33 | 342,57 | 388,66 | |
| 0,50 | 89,26 | 69,78 | |
| 0,75 | 19,36 | 0,00 | |
| 1,00 | 6,81 | 0,00 | |
| 1,25 | 9,30 | 0,00 | |
| 1,50 | 0,00 | 0,00 | |
| 1,75 | 0,00 | 0,00 | |
| 2,00 | 0,00 | 0,00 | |
| 2,50 | | | |

Les cinétiques d'absorption de l'acide gamma hydroxybutyrique (% Dose et vitesse) présentent les mêmes profils et sont superposables.

Les microgranules IR n'induisent pas de retard ni de modification du processus d' absorption de l'acide gamma hydroxybutyrique par rapport à la solution référence Alcover. La dissolution *in vivo* dans l'estomac est très rapide et totale.

### Dosage 2250 mg :Formulation microgranules versus Alcover

| | | |
|---|---|---|
| ETUDE PK SMO032/10/01 | | **PK STUDY SMO032/10/01 RAPPORT MOYEN D'ABSORPTION ORALE** |
| RAPPORT MOYEN D'ABSORPTION ORALE | | **MICRO-GR. IR B001 Lot 4SMOI002 VERSUS ALCOVER 2250 mg** |
| | | 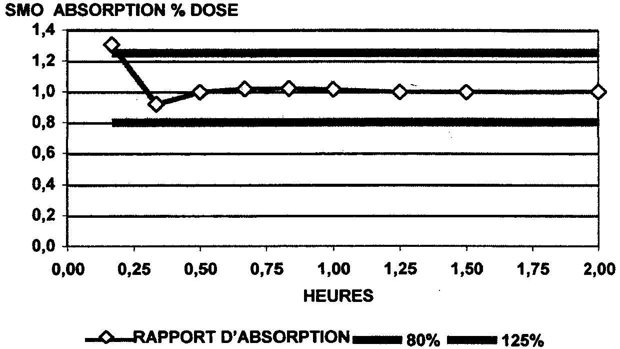 |
| MICRO-GR. IR B002 Batch 4SMOI002 | | |
| VERSUS ALCOVER 2250mg | | |
| HEURES | MICROG-IR RAPPORT D'ABSORPTION | |
| 0 | | |
| 0,17 | 1,30 | |
| 0,33 | 0,91 | |
| 0,50 | 1,00 | |
| 0,67 | 1,02 | |
| 0,83 | 1,02 | |
| 1,00 | 1,01 | |
| 1,25 | 0,99 | |
| 1,50 | 1,00 | |
| 2,00 | 1,00 | |

Le rapport R des profils d'absorption des microgranules IR 2250 mg et de la solution Alcover® est inclus entre les valeurs 0,8 et 1,2

Les rapports R des profils d'absorption cumulatives moyens de l'acide gamma hydroxybutyrique des microgranules et des solutions ont été établis à chaque temps entre les microgranules et les solutions administrées à la même dose : R(t) = % dose(t) après Microgranule divisé par % Dose(t) après solution

Les valeurs de ces rapports sont bien comprises dans l'intervalle 0,8 - 1,2 pendant la totalité du processus d'absorption (1,0 à 1,23 et 0,96 à 1,0) pour les doses 1750 mg ; elles sont comprises entre 1,3 et 0,91 pour les deux dosages 1750 et 2250 mg indiquant une absorption légèrement plus rapide des microgranules aux tous premier temps de la cinétique.

L'absorption orale de l'acide gamma hydroxybutyrique n'est pas dépendante du process de dissolution des microgranules du lot 4SMOI001 et du lot 4SMOI002.

Les résultats obtenus *in vivo* sont parfaitement corrélés avec les résultats observés *in vitro* qui indiquent très bien une dissolution extrêmement rapide (moins de 5 minutes) ne pouvant pas influencer l'absorption intestinale de l'acide gamma hydroxybutyrique. La méthode est donc parfaitement prédictive.

### V - Etude de la dissolution instantanée et cumulée des formulations selon l'invention

Afin d'étudier le comportement *in vivo* de ces formulations, leur dissolution instantanée a été évaluée. Ainsi, le pourcentage de principe actif libéré a été dosé à un temps t, ce qui permet de simuler le comportement *in vivo* de la composition dans le fluide déterminé.

La cinétique de dissolution cumulée sera à comparer avec l'évolution du taux plasmatique du principe actif en fonction du temps.

L'impact de la membrane d'enrobage a été étudié en comparant donc les dissolutions différentielles entre une forme immédiate et une forme enrobée.

L'effet recherché est ici uniquement de ralentir la diffusion du principe actif dans l'estomac afin de maintenir une absorption préférentielle dans les zones hautes du tube digestif.

Ainsi, il est également intéressant de disposer de formulations avec une dissolution à un taux de 80% de libération en deux heures.

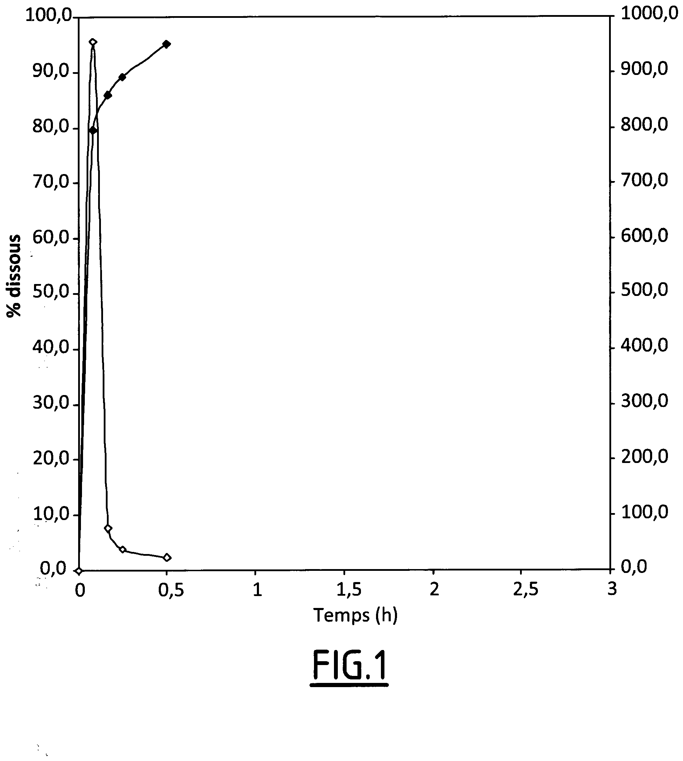

Les résultats de l'étude de la dissolution instantanée et cumulée des formulations à libération immédiate sont représentés sur la Figure 1. La courbe avec les losanges noirs correspond au pourcentage dissous de principe actif (%) en fonction du temps (en heures) et la courbe avec les losanges blancs correspond à la vitesse de dissolution pour la formulation de l'exemple 4.

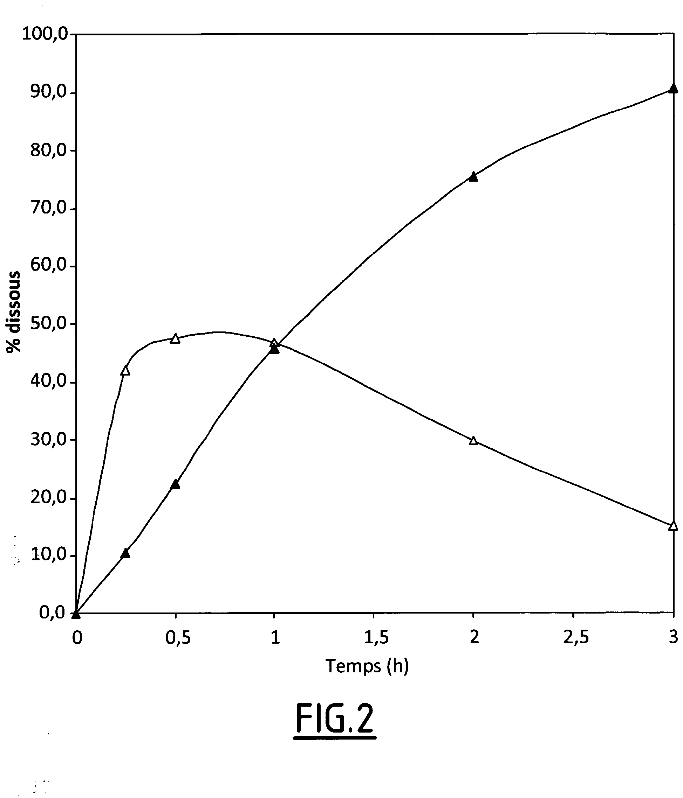

Les résultats de l'étude de la dissolution instantanée et cumulée des formulations à libération retardée sont représentés sur la Figure 2. La courbe avec les triangles noirs correspond au pourcentage dissous de principe actif (%) en fonction du temps (en heures) et la courbe avec les triangles blancs correspond à la vitesse de dissolution pour la formulation de l'exemple 5.

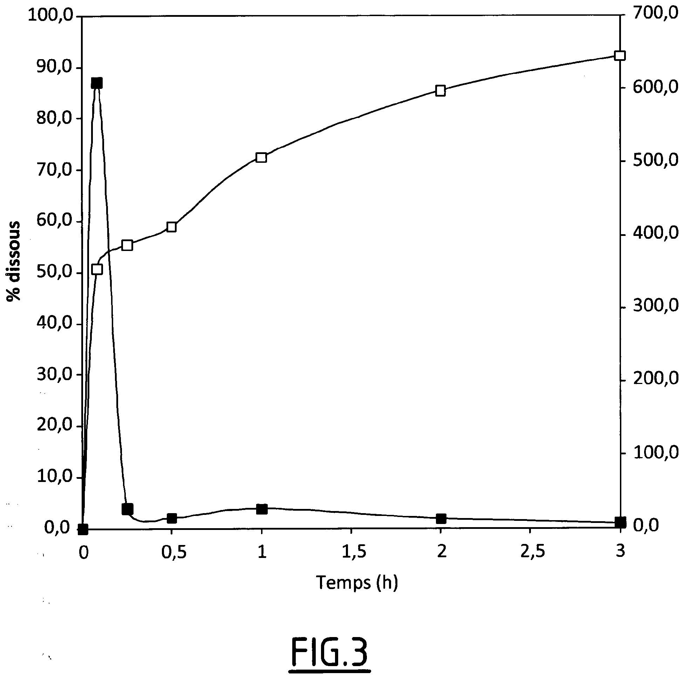

Les résultats de l'étude de la dissolution instantanée et cumulée des mélanges de formes à libération immédiate et libération retardée sont représentés sur la Figure 3. La courbe avec les carrés blancs correspond au pourcentage dissous de principe actif (%) en fonction du temps (en heures) et la courbe avec les carrés noirs correspond à la vitesse de dissolution pour la formulation de l'exemple 3.

### VI - Etudes cliniques

Deux études ont été menées chez l'homme :
1. 1^{ère} étude : formulations SMO.IR et SMO.SR contre 2 références (Réf.1 = Alcover® ; Réf. 2 = Xyrem®), administration de 1,75 g sur 12 volontaires mâles sains, à jeun.
2. 2^{ème} étude : formulation SMO.MR, administrée à 3,0 g, contre 1 référence (Réf. = Xyrem®) administrée à 2,25 g, sur 8 volontaires mâles sains, à jeun.

Les paramètres extraits de ces 2 études sont reportés ci-après.

**Résultats de la première étude**

| Formule | C max (µg/ml) (moyenne ± SD) | t max (h) (moyenne) | AUC (µg/ml.h) (moyenne ± SD) | t_{1/2} (h) (moyenne ± SD) |
|---|---|---|---|---|
| Réf. 1 (Alcover®) | 50,19 ± 15,27 | 0,50 | 61 ± 31 | 0,55 ± 0,14 |
| Réf. 2 (Xyrem®) | 55,37 ± 16,45 | 0,50 | 64 ± 31 | 0,57 ±0,21 |
| SMO.IR (exemple 1) | 54,47 ± 15,75 | 0,50 | 70 ± 46 | 0,56 ± 026 |
| SMO.SR (exemple 2) | 31,35 ± 20,67 | 1,25 | 52 ± 39 | 0,56 ± 026 |

**Résultats de la deuxième étude**

| Formule | C max (µg/ml) (moyenne ± SD) | t max (h) (moyenne) | AUC (µg/ml.h) (moyenne ± SD) | t_{1/2} (h) (moyenne ± SD) |
|---|---|---|---|---|
| Réf. (Xyrem®) | 61,95 ± 14,21 | 0,50 | 83 ±30 | 0,53 ± 0,11 |
| SMO.MR (exemple 3) | 68,52 ± 12,13 | 0,50 | 121 ± 54 | 0,62 ± 0,22 |

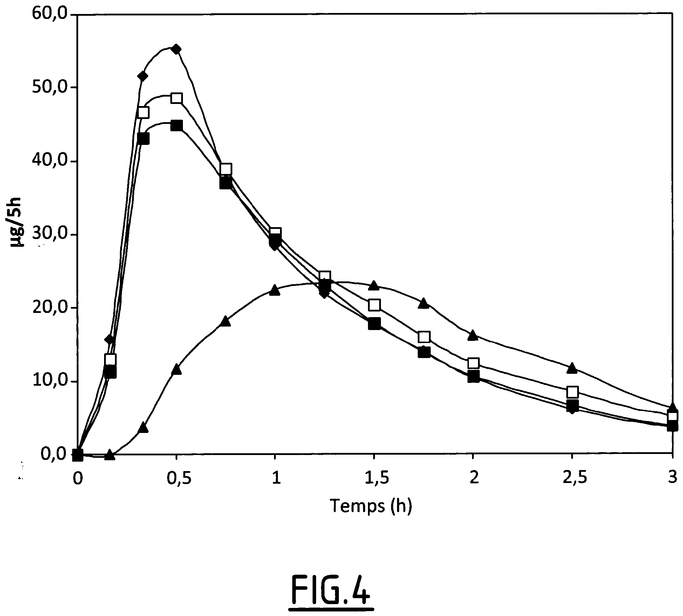

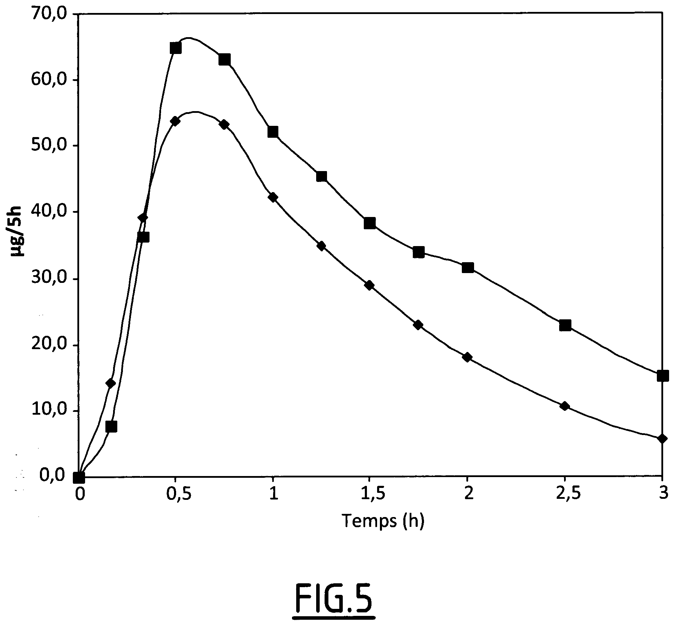

Les courbes de dissolution de ces deux études sont respectivement représentées sur les Figures 4 et 5. Ces figures représentent les quantités de principe actif dissous (en µg/h) en fonction du temps (h).

Dans la Figure 4, la courbe avec les carrés noirs correspond à la Réf.1, la courbe avec les losanges noirs correspond à la Réf.2, la courbe avec les carrés blancs correspond à la formulation de l'exemple 1 et la courbe avec les triangles noirs correspond à la formulation de l'exemple 2.

Dans la Figure 5, la courbe avec les losanges noirs correspond à la Réf. (Xyrem) et la courbe avec les carrés noirs correspond à la formulation de l'exemple 3.

La formulation immédiate solide testée présente des paramètres pharmacocinétiques équivalents aux deux formes orales liquides testées ; on obtient une bioéquivalence entre la formulation testée et la référence 1.

On remarque également pour les formulations immédiates que les parties ascendantes (absorption) et descendantes (élimination) sont équivalentes, ce qui laisse supposer un phénomène linéaire.

## Revendications

1. Granulé comprenant un coeur solide sur lequel est supporté un principe actif, ledit principe actif étant choisi parmi l'acide gamma-hydroxybutyrique ou l'un de ses sels pharmaceutiquement acceptables, ledit granulé étant **caractérisé en ce qu'**il se compose de :
- 15-25% de coeur solide,
- 50-60% de principe actif
- 5-15% de bicarbonate de sodium, générateur de gaz
- 2-18% d'aluminometasilicate de magnésium, diluant
- 3-10% de gomme laque, liant
- 3-6% de membrane d'enrobage

2. Granulé selon la revendication 1, **caractérisé en ce que** ledit coeur solide est choisi dans le groupe constitué des polyols, des gommes, des dérivés de la silice, des dérivés de calcium ou de potassium, des composés minéraux tels que les phosphates dicalciques, des phosphates tricalciques et des carbonates de calcium, du saccharose, des dérivés de cellulose, notamment de la cellulose microcristalline, de l'éthylcellulose et de l'hydroxypropylméthylcellulose, de l'amidon, et des mélanges de ceux-ci.

3. Granulé selon l'une des revendications 1 ou 2, **caractérisé en ce que** la membrane est constituée d'excipients d'enrobage pour libération immédiate.

4. Granulé selon l'une des revendications 1 ou 2, **caractérisé en ce que** la membrane est constituée d'excipients d'enrobage pour libération étalée.

5. Granulé selon l'une quelconque des revendications 1 à 4 **caractérisé en ce qu'**il comprend en outre un agent colorant.

6. Granulé selon l'une quelconque des revendications 1 à 5 **caractérisé en ce qu'**il comprend en outre un édulcorant.

7. Granulé selon l'une quelconque des revendications 1 à 6 **caractérisé en ce qu'**il comprend en outre un agent aromatisant.

8. Composition pharmaceutique comprenant un mélange de granulés selon l'une quelconque des revendications 1 à 7, dans laquelle ledit mélange est constitué de deux groupes de granulés (A) et (B), les granulés (A) et les granulés (B) présentant des cinétiques différentes de libération du principe actif.

9. Granulé selon l'une quelconque des revendications 1 à 7 pour l'utilisation dans le traitement de pathologies nécessitant au moins la mise à disposition *in vivo* très rapide du GHB.

10. Granulé l'une quelconque des revendications 1 à 7 pour l'utilisation dans le traitement selon la revendication 9 **caractérisé en ce que** la pathologie est la narcolepsie.

11. Granulé l'une quelconque des revendications 1 à 7 pour l'utilisation dans le traitement selon la revendication 9 **caractérisé en ce que** la pathologie est le sevrage de l'alcoolisme.

## Patentansprüche

1. Granulat, umfassend einen festen Kern, auf dem ein Wirkstoff getragen wird, wobei der Wirkstoff ausgewählt ist aus Gamma-Hydroxybuttersäure oder einem ihrer pharmazeutisch annehmbaren Salze, wobei das Granulat **dadurch gekennzeichnet ist, dass** es aus Folgendem besteht:
- 15 - 25 % festem Kern,
- 50 - 60 % Wirkstoff
- 5 - 15 % Natriumbicarbonat, Gasgenerator
- 2 - 18 % Magnesium-Aluminometasilikat, Verdünnungsmittel
- 3 - 10 % Schellack, Bindemittel
- 3 - 6 % umhüllende Membran.

2. Granulat nach Anspruch 1, **dadurch gekennzeichnet, dass** das feste Granulat ausgewählt ist aus der Gruppe, bestehend aus Polyolen, Gummi, Siliciumderivaten, Calcium- oder Kaliumderivaten, mineralischen Zersetzungsprodukten wie z. B. Dicalciumphosphaten, Tricalciumphosphaten und Calciumcarbonaten, Saccharose, Zellulosederivate, insbesondere mikrokristalliner Cellulose, Ethylcellulose und Hydroxypropylmethylcellulose, Stärke und Mischungen derselben.

3. Granulat nach einem der Ansprüche 1 oder 2, **dadurch gekennzeichnet, dass** die Membran aus umhüllenden Trägerstoffen zur unmittelbaren Freisetzung besteht.

4. Granulat nach einem der Ansprüche 1 oder 2, **dadurch gekennzeichnet, dass** die Membran aus umhüllenden Trägerstoffen zur gestaffelten Freisetzung besteht.

5. Granulat nach einem der Ansprüche 1 bis 4, **dadurch gekennzeichnet, dass** es außerdem ein Farbmittel umfasst.

6. Granulat nach einem der Ansprüche 1 bis 5, **dadurch gekennzeichnet, dass** es außerdem einen Süßstoff umfasst.

7. Granulat nach einem beliebigen der Ansprüche 1 bis 6, **dadurch gekennzeichnet, dass** es außerdem einen Geschmacksstoff umfasst.

8. Pharmazeutische Zusammensetzung, umfassend eine Mischung aus Granulaten nach einem beliebigen der Ansprüche 1 bis 7, wobei die Mischung aus zwei Gruppen von Granulaten (A) und (B) besteht, wobei die Granulate (A) und die Granulate (B) verschiedene Kinetiken zur Freigabe des Wirkstoffs umfassen.

9. Granulat nach einem beliebigen der Ansprüche 1 bis 7 zur Verwendung bei der Behandlung von Pathologien, die mindestens die sehr schnelle *in vivo*-Bereitstellung des GHB erfordern.

10. Granulat nach einem beliebigen der Ansprüche 1 bis 7 zur Verwendung bei der Behandlung nach Anspruch 9, **dadurch gekennzeichnet, dass** die Pathologie die Narkolepsie ist.

11. Granulat nach einem beliebigen der Ansprüche 1 bis 7 zur Verwendung bei der Behandlung nach Anspruch 9, **dadurch gekennzeichnet, dass** die Pathologie der Alkoholentzug ist.

## Claims

1. Granule comprising a solid core carrying an active ingredient, said active ingredient being selected from among gamma-hydroxy butyric acid or one of the pharmaceutically acceptable salts thereof, said granule **characterized in that** it is composed of:
- 15-25 % solid core,
- 50-60 % active ingredient;
- 5-15 % sodium bicarbonate, gas generator;
- 2-18 % magnesium aluminometasilicate, diluent;
- 3-10 % shellac, binder;
- 3-6 % coating membrane.

2. The granule according to claim 1, wherein the solid core is selected from the group formed by polyols, gums, silica derivatives, calcium or potassium derivatives, mineral compounds such as dicalcium phosphates, tricalcium phosphates and calcium carbonates, sucrose, cellulose derivatives in particular microcrystalline cellulose, ethyl cellulose and hydroxypropyl methylcellulose, starch and mixtures thereof.

3. The granule according to any of claims 1 to 2, wherein the membrane is composed of coating excipients for immediate release.

4. The granule according to any of claims 1 to 2, wherein the membrane is composed of coating excipients for sustained release.

5. The granule according to any of claims 1 to 4, **characterized in that** it further comprises a colouring agent.

6. The granule according to any of claims 1 to 5, **characterized in that** it further comprises a sweetener.

7. The granule according to any of claims 1 to 6, **characterized in that** it further comprises a flavouring agent.

8. Pharmaceutical composition comprising a mixture of granules according to any of claims 1 to 7, wherein said mixture is composed of two groups of granules (A) and (B), granules (A) and granules (B) having different release kinetics of active ingredient.

9. Application of the granule according to any of claims 1 to 7 for use in the treatment of pathologies requiring at least very rapid *in vivo* availability of GHB.

10. Application of the granule according any of claims 1 to 7 for use in the treatment according to claim 9, **characterized in that** the pathology is narcolepsy.

11. Application of the granule according any of claims 1 to 7 for use in the treatment according to claim 9, **characterized in that** the pathology is alcohol withdrawal.
